# EUROPEAN PATENT APPLICATION

(11) **EP 2 484 382 A1**
(43) Date of publication of application: **08.08.2012**
(21) Application number: 12154081.9
(22) Date of filing: 30.03.2006
(51) Int. Cl.: A61K 45/06, A61K 45/00, A61P 11/00, A61K 9/00, C07D 413/14, A61K 31/4709, A61K 9/12

(54) **Medicament comprising a phosphodiesterase IV inhibitor in an inhalable form**

(30) Priority: 30.03.2005 US 666420 P; 08.11.2005 US 734452 P; 29.03.2006 US 786960 P
(62) Divisional of application: 06740200.8
(71) Applicant: Schering Corporation, Kenilworth, NJ 07033 (US)
(72) Inventor: Sequeira, Joel, A., Ocean, NJ New Jersey 07712 (US); Yang, Tsong-Toh, Warren, NJ New Jersey 07059 (US)
(74) Representative: Simpson, Tobias Rutger

(57) **Abstract**

Disclosed are inhalable medicaments and methods based on an anticholinergic in combination with a corticosteroid, and a long acting beta agonist, for simultaneous or sequential administration in the prevention or treatment of a respiratory, inflammatory or obstructive airway disease. In addition, disclosed are inhalable medicaments and methods based on combinations of an anticholinergic and a corticosteroid; an anticholinergic and a long acting beta agonist; or a corticosteroid and a long acting beta agonist, for simultaneous or sequential administration in the prevention or treatment of a respiratory, inflammatory or obstructive airway disease. Also disclosed are inhalable medicaments and methods comprising a phosphodiesterase IV inhibitor for administration in the prevention or treatment of a respiratory, inflammatory or obstructive airway disease.

## Description

### FIELD OF THE INVENTION

The present invention relates to novel inhalable medicaments and methods based on an anticholinergic in combination with a corticosteroid, and a long acting beta agonist, for simultaneous or sequential administration in the prevention or treatment of a respiratory, inflammatory or obstructive airway disease. In addition, the present invention relates to novel inhalable medicaments and methods based on combinations of an anticholinergic and a corticosteroid; an anticholinergic and a long acting beta agonist; or a corticosteroid and a long acting beta agonist, for simultaneous or sequential administration in the prevention or treatment of a respiratory, inflammatory or obstructive airway disease. The present invention also relates to novel inhalable medicaments and methods comprising a phosphodiesterase IV inhibitor for administration in the prevention or treatment of a respiratory, inflammatory or obstructive airway disease..

### SUMMARY OF THE INVENTION

Accordingly, there is disclosed a medicament comprising, separately or together, (A) an anticholinergic, (B) a corticosteroid, and (C) a long acting beta agonist, for simultaneous or sequential administration in the prevention or treatment of a respiratory, inflammatory or obstructive airway disease, each of (A), (B), and (C) being in an inhalable form.

In one embodiment, the medicament is a pharmaceutical composition comprising an inhalable mixture of effective amounts of (A) and (B) and (C), optionally together with a pharmaceutically acceptable carrier. Preferably, the medicament is an aerosol comprising a mixture of (A) and (B) and (C) in solution or dispersion in a propellant, or a combination of an aerosol containing (A) in solution or dispersion in a propellant with an aerosol containing (B) in solution or dispersion in a propellant with an aerosol containing (C) in solution or dispersion in a propellant. More preferably, (A) or (B) or (C), or (A) and (B) and (C), are in dispersion in the propellant, which is a halogen-substituted hydrocarbon. More preferably, (A) or (B) or (C), or each of (A) and (B) and (C), has an average particle diameter of up to 10 microns.

In another embodiment, the medicament is a nebulizable composition comprising a dispersion of (A) and (B) and (C) in an aqueous, organic or aqueous/organic medium or a combination of a dispersion of (A) in said medium with a dispersion of (B) in said medium with a dispersion of (C) in said medium.

In one embodiment, the medicament is a dry powder comprising finely divided (A) or (B) or (C), or finely divided (A) and (B) and (C), optionally together with a pharmaceutically acceptable carrier in finely divided form. Preferably, the carrier is present and is a saccharide. More preferably, the carrier is lactose. Preferably, (A) or (B) or (C), or each of (A) and (B) and (C), has an average particle diameter up to 10 microns.

There is also disclosed a medicament comprising, separately or together, (A) an anticholinergic, (B) a corticosteroid, and (C) a long acting beta agonist, for simultaneous or sequential administration in the prevention or treatment of a respiratory, inflammatory or obstructive airway disease, each of (A), (B), and (C) being in inhalable form, wherein the anticholinergic is (R)-3-[2-hydroxy-2,2-(dithien-2-yl)acetoxy]-1-1 [2-(phenyl)ethyl]-1-azoniabicyclo[2.2.2] octane or a pharmaceutically acceptable salt or hydrate thereof, wherein the corticosteroid is Mometasone Furoate or a pharmaceutically acceptable salt or hydrate thereof, and wherein the long acting beta agonist is Carmoterol, Indacaterol or a pharmaceutically acceptable salt or hydrate of any of the above, or a combination of two or more of the above.

Likewise, there is disclosed a medicament comprising, separately or together, (A) an anticholinergic, (B) a corticosteroid, and (C) a long acting beta agonist, for simultaneous or sequential administration in the prevention or treatment of a respiratory, inflammatory or obstructive airway disease, each of (A), (B), and (C) being in inhalable form, wherein the anticholinergic is Glycopyrrolate or a pharmaceutically acceptable salt or hydrate thereof, wherein the corticosteroid is Mometasone Furoate or a pharmaceutically acceptable salt or hydrate thereof, and wherein the long acting beta agonist is Carmoterol, Indacaterol or a pharmaceutically acceptable salt or hydrate of any of the above, or a combination of two or more of the above.

In one embodiment, the anticholinergic is (R)-3-[2-hydroxy-2,2-(dithien-2-yl)acetoxy]-1-1 [2-(phenyl)ethyl]-1-azoniabicyclo[2.2.2] octane, Glycopyrrolate, Ipratropium Bromide, Oxitropium Bromide, Atropine Methyl Nitrate, Atropine Sulfate, Ipratropium, Belladonna Extract, Scopolamine, Scopolamine Methobromide, Methscopolamine, Homatropine Methobromide, Hyoscyamine, Isopriopramide, Orphenadrine, Benzalkonium Chloride, Tiotropium Bromide, GSK202405, or a pharmaceutically acceptable salt or hydrate of any of the above, or a combination of two or more of the above. In one preferred embodiment, the anticholinergic is (R)-3-[2-hydroxy-2,2-(dithien-2-yl)acetoxy]-1-1 [2-(phenyl)ethyl]-1-azoniabicyclo[2.2.2] octane or a pharmaceutically acceptable salt or hydrate thereof. In another preferred embodiment, the anticholinergic is Glycopyrrolate or a pharmaceutically acceptable salt or hydrate thereof.

In one embodiment, the corticosteroid is Mometasone Furoate; Beclomethasone Dipropionate; Budesonide; Fluticasone; Dexamethasone; Flunisolide; Triamcinolone; (22R)-6.alpha.,9.alpha.-difluoro-11.beta.,21-dihydroxy-16.alpha.,17.alpha. -propylmethylenedioxy-4-pregnen-3,20-dione, Tipredane, GSK685698, GSK799943 or a pharmaceutically acceptable salt or hydrate of any of the above, or a combination of two or more of the above.

In one preferred embodiment, the anticholinergic is (R)-3-[2-hydroxy-2,2-(dithien-2-yl)acetoxy]-1-1 [2-(phenyl)ethyl]-1-azoniabicyclo[2.2.2] octane or a pharmaceutically acceptable salt or hydrate thereof and the corticosteroid is Mometasone Furoate or a pharmaceutically acceptable salt or hydrate thereof. In another preferred embodiment, the anticholinergic is Glycopyrrolate or a pharmaceutically acceptable salt or hydrate thereof and the corticosteroid is Mometasone Furoate or a pharmaceutically acceptable salt or hydrate thereof.

In one embodiment, the long acting beta agonist is Carmoterol, Indacaterol, TA-2005, Albuterol, Terbutaline, Salmeterol, Bitolterol, Formoterol, Fenoterol, Metaprotenerol, GSK159802, GSK642444, GSK159797, GSK597901, GSK678077, or a pharmaceutically acceptable salt or hydrate of any of the above, or a combination of two or more of the above. In one preferred embodiment, the long acting beta agonist is Carmoterol or a pharmaceutically acceptable salt or hydrate thereof. In another preferred embodiment, the long acting beta agonist is Indacaterol or a pharmaceutically acceptable salt or hydrate thereof.

In one preferred embodiment, the long acting beta agonist is Carmoterol or a pharmaceutically acceptable salt or hydrate thereof and the corticosteroid is Mometasone Furoate or a pharmaceutically acceptable salt or hydrate thereof. In another preferred embodiment, the long acting beta agonist is Indacaterol or a pharmaceutically acceptable salt or hydrate thereof and the corticosteroid is Mometasone Furoate or a pharmaceutically acceptable salt or hydrate thereof.

In one embodiment, the medicament comprising (A), (B), and (C) as defined above, is in admixture with a polyanionic beta-cyclodextrin derivative with about one to about seven sodium sulfonate groups separated from the lipophilic cavity of the polyanionic beta-cyclodextrin derivative by at least one butyl ether spacer group.

In another embodiment, the medicament further comprises, (D) a phosphodiesterase IV inhibitor, wherein (D) is in an inhalable form. In one preferred embodiment, the phosphodiesterase IV inhibitor is Cilomilast, Roflumilast, Tetomilast, 1-[[5-(1 (S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester or a pharmaceutically acceptable salt or hydrate of any of the above, or a combination of two or more of the above. Preferably, the phosphodiesterase IV inhibitor is 1-[[5-(1(S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester or a pharmaceutically acceptable salt or hydrate thereof. More preferably, the phosphodiesterase IV inhibitor is a xinafoate salt of 1-[[5-(1 (S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester.

In one embodiment, the anticholinergic is Glycopyrrolate or a pharmaceutically acceptable salt or hydrate thereof, wherein the corticosteroid is Mometasone Furoate or a pharmaceutically acceptable salt or hydrate thereof, wherein the long acting beta agonist is Carmoterol, Indacaterol or a pharmaceutically acceptable salt or hydrate of any of the above, or a combination of two or more of the above, and wherein the phosphodiesterase IV inhibitor is a xinafoate salt of 1-[[5-(1(S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester.

In another embodiment, the anticholinergic is (R)-3-[2-hydroxy-2,2-(dithien-2-yl)acetoxy]-1-1 [2-(phenyl)ethyl]-1-azoniabicyclo[2.2.2] octane or a pharmaceutically acceptable salt or hydrate thereof, wherein the corticosteroid is Mometasone Furoate or a pharmaceutically acceptable salt or hydrate thereof, wherein the long acting beta agonist is Carmoterol, Indacaterol or a pharmaceutically acceptable salt or hydrate of any of the above, or a combination of two or more of the above, and wherein the phosphodiesterase IV inhibitor is a xinafoate salt of 1-[[5-(1 (S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester.

In one embodiment, the medicament comprising (A), (B), (C), and (D) as defined above, is in admixture with a polyanionic beta-cyclodextrin derivative with about one to about seven sodium sulfonate groups separated from the lipophilic cavity of the polyanionic beta-cyclodextrin derivative by at least one butyl ether spacer group.

There is also disclosed a medicament comprising phosphodiesterase IV inhibitor in an inhalable form. In one preferred embodiment, the phosphodiesterase IV inhibitor is Cilomilast, Roflumilast, Tetomilast, 1-[[5-(1 (S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester or a pharmaceutically acceptable salt or hydrate of any of the above, or a combination of two or more of the above. Preferably, the phosphodiesterase IV inhibitor is 1-[[5-(1 (S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester or a pharmaceutically acceptable salt or hydrate thereof. More preferably, the phosphodiesterase IV inhibitor is a xinafoate salt of 1-[[5-(1 (S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester.

In one embodiment, the medicament comprising phosphodiesterase IV inhibitor in an inhalable form further comprises separately or together, one or more of the following: (A) an anticholinergic in an inhalable form, (B) a corticosteroid in an inhalable form, (C) a long acting beta agonist in an inhalable form, for simultaneous or sequential administration in the prevention or treatment of a respiratory, inflammatory or obstructive airway disease.

There is also disclosed a pharmaceutical kit comprising (A), (B), and (C) as defined above, in separate unit dosage forms, said forms being suitable for administration of (A) and (B) and (C) in effective amounts, together with one or more inhalation devices for administration of (A) and (B) and (C), and instructions for administering (A) and (B) and (C).

There is also disclosed a pharmaceutical kit comprising (A), (B), (C) and (D) as defined above, in separate unit dosage forms, said forms being suitable for administration of (A) and (B) and (C) and (D) in effective amounts, together with one or more inhalation devices for administration of (A) and (B) and (C) and (D), and instructions for administering (A) and (B) and (C) and (D).

In addition, there is disclosed a medicament comprising, separately or together, (A) an anticholinergic and (B) a corticosteroid, for simultaneous or sequential administration in the prevention or treatment of a respiratory, inflammatory or obstructive airway disease, each of (A) and (B) being in an inhalable form. In one embodiment, the anticholinergic is (R)-3-[2-hydroxy-2,2-(dithien-2-yl)acetoxy]-1-1[2-(phenyl)ethyl]-1-azoniabicyclo[2.2.2] octane, Glycopyrrolate or a pharmaceutically acceptable salt or hydrate of any of the above, or a combination of two or more of the above, and the corticosteroid is Mometasone Furoate or a pharmaceutically acceptable salt or hydrate thereof.

In addition, there is disclosed a medicament comprising, separately or together, (A) an anticholinergic and (C) a long acting beta agonist, for simultaneous or sequential administration in the prevention or treatment of a respiratory, inflammatory or obstructive airway disease, each of (A) and (C) being in an inhalable form. In one embodiment, the anticholinergic is (R)-3-[2-hydroxy-2,2-(dithien-2-yl)acetoxy]-1-1 [2-(phenyl)ethyl]-1-azoniabicyclo[2.2.2] octane or a pharmaceutically acceptable salt or hydrate thereof, and the long acting beta agonist is Carmoterol, Indacaterol or a pharmaceutically acceptable salt or hydrate of any of the above, or a combination of two or more of the above. In another embodiment, the anticholinergic is Glycopyrrolate or a pharmaceutically acceptable salt or hydrate thereof, and the long acting beta agonist is Carmoterol, Indacaterol or a pharmaceutically acceptable salt or hydrate of any of the above, or a combination of two or more of the above.

In addition, there is disclosed a medicament comprising, separately or together, (B) a corticosteroid and (C) a long acting beta agonist, for simultaneous or sequential administration in the prevention or treatment of a respiratory, inflammatory or obstructive airway disease, each of (B) and (C) being in an inhalable form. In one embodiment, the corticosteroid is Mometasone Furoate or a pharmaceutically acceptable salt or hydrate thereof, and the long acting beta agonist is Carmoterol, Indacaterol or a pharmaceutically acceptable salt or hydrate of any of the above, or a combination of two or more of the above.

In one embodiment, the medicament is a pharmaceutical composition comprising an inhalable mixture of effective amounts of (A) and (B); (A) and (C); or (B) and C), optionally together with a pharmaceutically acceptable carrier.

The pharmaceutical compositions and medicaments may be administered prophylactically as a preventative or during the course of a medical condition as a treatment or cure.

### DETAILED DESCRIPTION OF THE INVENTION

Most preferably, in accordance with the present invention, the medicament is or contains a material capable of being administered in an inhalable dry powder form to the respiratory system, including the lungs. For example, a medicament in accordance with the present invention could be administered so that it is absorbed into the blood stream through the lungs. More preferably, however, the medicament is a powdered drug in dry powder form which is effective to treat some condition of the lungs or respiratory system directly and/or topically.

Particularly preferred corticosteroids in accordance with the present invention include, without limitation, Mometasone Furoate; Beclomethasone Dipropionate; Budesonide; Fluticasone; Dexamethasone; Flunisolide; Triamcinolone; (22R)-6.alpha.,9.alpha.-difluoro-11.beta.,21-dihydroxy-16.alpha.,17.alpha. -propylmethylenedioxy-4-pregnen-3,20-dione, Tipredane, GSK685698, GSK799943, or a pharmaceutically acceptable salt or hydrate of any of the above, or a combination of two or more of the above.

The corticosteroid preferably for use in the present invention is preferably Mometasone Furoate, the active agent of Nasonex® and Asmanex®. These products are available from Schering-Plough Corporation, Kenilworth, New Jersey. It is an anti-inflammatory corticosteroid having the chemical name, 9, 21-Dichloro-11 (beta), 17-dihydroxy-16(alpha)-methylpregna-1,4-diene-3,20-dione 17-(2 Furoate). This active agent may be present in an amount of about 25 to about 1000 µg per actuation of an MDI or DPI. Doses of 25 µg, 50 µg, 75 µg, 100 µg, 125 µg, 150 µg, 175 µg, 200 µg, 250 µg, 300 µg, 400 µg and/or 500 µg are preferred.

Other inhalable corticosteroids for use in the present invention include GSK685698 (also known as GW686698X, and under the trademark Avamys® or Allernmist®) and GSK799943 available from GlaxoSmithKline.

A preferred anticholinergic is known as LAS 34273 (chemically identified as (R)-3-[2-hydroxy-2,2-(dithien-2-yl)acetoxy]-1-1 [2-(phenyl)ethyl]-1-azoniabicyclo[2.2.2]octane) available from Almirall Prodesfarma SA. It may be prepared in accordance with the procedures set forth in WO 01/04118 and U.S. Patent No. 6,750,226, both of which are incorporated by reference. It is a new long acting anticholinergic with once a day efficacy. This active agent may be present in an amount of about 10 to about 1000 µg per day, preferably in an amount of about 100 to about 500 µg per day.

Glycopyrrolate, also known as glycopyrronium bromide is an anticholinergic drug that decreases tracheobronchial and pharyngeal secretions. It does not easily cross the blood-brain barrier, like atropine and scopolamine, and thus it has less CNS side effects. This active agent may be present in an amount of about 25 µg to about 1000 µg per actuation of an MDI or DPI.

The long acting beta agonist can be one of a number of different pharmaceutically active agents. By long acting it is meant that the drug will have an effect on the bronchi that lasts around 6 hours or more, up to about 12 hours in some instances and up to about 24 hours in other instances. This active agent may be present in an amount of about 10 to about 1000 µg per day.

Long acting beta agonists for use in the present invention include without limitation, Carmoterol, Indacterol, TA-2005, Albuterol, Terbutaline, Salmeterol, Bitolterol, Formoterol, Fenoterol, Metaprotenerol, GSK159802, GSK642444, GSK159797, GSK597901, GSK678077, or any one of those described in the following patents which are incorporated by reference in their entirety: 6,949,568, 6,919,482, 6,916,961, 6,713,651, 6,683,115, 6,670,376, 6,653,323, 6,593,497, 6,576,793 and 6,541,669, all of which are assigned to Theravance Inc., of California, or a pharmaceutically acceptable salt or hydrate of any of the above, or a combination of two or more of the above.

Carmoterol, also known as Karmoterolis and Carmoterolum, is identified chemically as 8-hydroxy-5-[(1R)-1-hydroxy-2-(4-methoxyphenyl)-1-methylethyl]amino)+ethyl]quinolin-2(1 H)-one. It is a new long acting beta agonist available from Chiesi Farmaceutici, Parma, Italy.

Formoterol (also known as eformoterol) e.g., as the fumarate or tartrate, a highly selective long-lasting Beta-adrenergic agonist having bronchospasmolytic effect, is effective in the treatment of reversible obstructive lung ailments of various genesis, particularly asthmatic conditions.

TA-2005, another long acting beta agonist, is chemically identified as 2(1H)-Quinolinone, 8-hydroxy-5-[1-hydroxy-2-[[2-(4-(methoxyphenyl)-1-methylethyl]amino]ethyl] -monohydrochloride, [R-(R*,R*)]-- also identified by Chemical Abstract Service Registry Number 137888-11-0 and disclosed in U.S. Pat. No. 4,579,854, the text of which is hereby incorporated by reference. Its chemical name is (±)-2-hydroxy-5-[(1RS)-1-hydroxy-2-[[(1 RS)-2-(4-methoxyphenyl)-1-methylethyl]- amino]ethyl]formanilide Fumarate dihydrate. This active agent may be present in an amount of about 3 to about 50 µg per actuation of the MDI or DPI. It is available under the trade name of Foradil®.

Another long acting selective beta₂ -adrenergic bronchodilator for use in the present invention is Indacaterol (also known as QAB149), reportedly useful for the treatment of asthma and chronic obstructive pulmonary disorder which is being developed by Novartis Corporation, East Hanover, New Jersey. This active agent may be present in an amount of about 10 µg to about 1000 µg per day.

Another long acting selective beta₂ -adrenergic bronchodilator for use in the present invention is Metaproterenol, which is the subject of U.S. Pat. No. 3,341,594 and is commercially available under the trade names of Alotec, Alupent, Metaprel or Novasmasol.

Another long acting selective beta ₂ -adrenergic bronchodilator for use in the present invention is Terbutaline, which is described in U.S. Pat. No. 3,938,838 and is available commercially as Brethine from Novartis. The preparation of fenoterol is described in U.S. Pat. No. 4,341,593. It is sold under several trade names, including Airum, Berotec, Dosberotec and Partusisten. Albuterol (also known as Salbutamol) is sold under the trademark Proventil® by Schering Corporation. Salmeterol is sold under the trademark Serevent®, available from GlaxoSmithKline.

Other active agents for use in another embodiment of the present invention include phosphodiesterase IV inhibitors. One such active agent is SB 207499 (c-4-cyano-4-(3-cyclopentyloxy-4-methoxy-phenyl)-r-L-cyclohexane carboxylic acid), available under the tradename Ariflo®. Ariflo, also known a Cilomilast, is an oral selective phosphodiesterase (PDE) IV inhibitor under development by GlaxoSmithKline Pharmaceuticals for treatment of COPD.

In another embodiment of the present invention, the compositions of the present invention may further include the phosphodiesterase IV inhibitor Roflumilast. Roflumilast is being developed by Altana Pharma as a potential treatment of asthma and COPD. It is awaiting regulatory approval in Europe and phase III clinical trials are ongoing in the US. Like Cilomilast, this active agent is also well tolerated, and does not appear to be associated with the nausea and vomiting that have posed a problem with older PDE-IV inhibitors. In a multicentre randomised study, the tolerability profile observed in 516 patients with COPD was equivalent to that seen in placebo recipients after 26 weeks of dosing. Altana said that Roflumilast may also have a role in the treatment of psoriasis and allergic rhinitis.

In another embodiment of the present invention, the compositions of the present invention may further include Tetomilast. Tetomilast (also known as OPC-6535) is a phosphodiesterase-4 inhibitor currently in phase II development with Otsuka in the U.S. as a treatment for COPD. Preclinical studies have demonstrated the efficacy of Tetomilast in the inhibition of airway inflammation in COPD, and in a guinea-pig model of COPD induced by cigarette smoke exposure, Tetomilast resulted in improvements in specific airway resistance and neutrophilia (O'Mahoney, IDrugs, 8(6):502-507 (2005).

According to one embodiment, all the active agents of the medicaments would be administered at the same time, or very close in time. Alternatively, one active agent could be taken in the morning and one later in the day. Or in another scenario, one active agent could be taken twice daily and the other once daily, either at the same time as one of the twice-a-day dosing occurred, or separately. Preferably all of the active agents would be taken together at the same time.

In one embodiment of the invention, the inhalable form of the medicament is a dry powder, i.e. (A) and/or (B) and/or (C) are present in a dry powder comprising finely divided (A) and (B) and (C) optionally together with a finely divided pharmaceutically acceptable carrier, which is preferably present and may be one or more materials known as pharmaceutically acceptable carriers, preferably chosen from materials known as carriers in dry powder inhalation compositions, for example saccharides, including monosaccharides, disaccharides, polysaccharides and sugar alcohols such as arabinose, glucose, fructose, ribose, mannose, sucrose, trehalose, lactose, maltose, starches, dextran or mannitol. An especially preferred carrier is lactose. The dry powder may be in capsules of gelatin or plastic, or in blisters, for use in a dry powder inhalation device, preferably in dosage units of (A) and/or (B) and/or (C) together with the carrier in amounts to bring the total weight of powder per capsule to from 5 mg to 50 mg. Alternatively, the dry powder may be contained as a reservoir in a multi-dose dry powder inhalation device. The amount of active agent(s) in a multi-dose dry powder inhalation device would be such that two actuations per day would deliver the requisite amount of active agent(s) per day.

In one embodiment, the medicaments of the present invention are prepared according to the methods of producing an agglomerate of drug and solid binder described in U.S. Patent No. 6,503,537, incorporated herein by reference in its entirety.

In one embodiment, the medicaments of the present invention are delivered using an inhaler as described in U.S. Patent No. 6,240,918, 5,687,710, or 5,829,434, incorporated herein by reference in their entirety.

In another embodiment, the medicaments of the present invention are suspended in a liquefied propellant as described in EP Patent Publication No. 1 420 759, incorporated herein by reference in its entirety.

In formulations of the present invention which are suitable for treating lower respiratory system disorders such as asthma, at least a substantial portion of the active agent is present as suspended particles having respirable sizes, e.g., about 0.5 to about 10 micrometers in their largest dimension. In formulations which are suitable for treating upper respiratory system disorders such as rhinitis, somewhat larger active agent particles may be permissible, but the foregoing size range remains preferred. Where the active agent forms a suspension, the particle size should be relatively uniform, with substantially all the particles preferably ranging between about 0.1-25 microns, preferably 0.5-10 microns, more preferably 1-5 microns. Particles larger than 25 microns may be held up in the oropharyngeal cavity, while particles smaller than about 0.5 micron preferably are not utilized, since they would be more likely to be exhaled and, therefore, not reach the lungs of the patient.

In another embodiment, the medicament may be administered via nebulization. The suspension formulations of the invention may be delivered to a patient using any of the usual nebulizer devices. Typical commercial nebulizer devices produce dispersions of droplets in gas streams by one of two methods. Jet nebulizers use a compressed air supply to draw up a fluid by venturi action and introduce it into a flowing gas stream, after which the fluid is caused to impact one or more stationary baffles to remove excessively large droplets. Ultrasonic nebulizers use an electrically driven transducer to subject a fluid to high-frequency oscillations, producing a cloud of droplets which can be entrained in a moving gas stream; these devices are less preferred for delivering suspensions. There are hand-held nebulizers which atomize the fluid with a squeeze bulb air supply, but the more widely used equipment incorporates an electrically powered compressor or connects to a cylinder of compressed gas. Although the various devices which are commercially available vary considerably in their delivery efficiency for a given medicament, they all are useful for the treatment of the present invention; it is necessary for the prescriber to specify an exact amount of medicament formulation which is to be charged to each particular device, since their respective outputs of respirable droplets are far from identical.

Suspension formulations suitable for nebulization must, of course, contain solid particles of a respirable size (e.g., preferably averaging less than about 5. µm in the largest dimension and more preferably averaging less than about 2 µm, and must maintain their suspended particle size distribution during storage. In addition, the particle-containing droplets formed during nebulization of the formulations must have appropriate sizes for deposition in the desired area of the respiratory system.

Since the formulations of the invention are to be inhaled, it is necessary that they be free of pathogenic organisms. Thus, they may be prepared and handled under sterile conditions, or may be sterilized before or after packaging. In addition, or in lieu of sterilization, a preservative may be incorporated to minimize the possibility of microbial contamination. In addition, all active agents of the formulations must be chosen for inhalation safety, as the treated tissues are quite sensitive to irritants; it is commonly known that many of the common preservatives have a considerable potential for causing irritation.

Water for use in the formulations should meet or exceed the applicable regulatory requirements for use in inhaled drugs. Specifications established by the United States Pharmacopoeia for "Sterile Water for Injection" or "Sterile Water for Inhalation" are examples of water suitable for use to prepare formulations of the invention.

Surfactants are frequently categorized by their chemical nature, i.e., as cationic, anionic or nonionic. Cationic surfactants, such as cetyl pyridinium chloride, and anionic surfactants, such as docusate sodium, do not appear to provide proper dispersions of particles in the nebulizable formulations.

Many nonionic surfactants are suitable for maintaining the particulate suspensions of the invention. These include surfactants identified as "polysorbates" in the CTFA International Cosmetic Ingredient Dictionary; such surfactants are mixtures of fatty acid esters (predominately monoesters) of sorbitol and sorbitol anhydrides, condensed with ethylene oxide. Although these surfactants vary widely in their hydrophilic-lipophilic balance ("HLB") numbers, they all appear to function well in the invention.

Commercially available polysorbates which are useful in the invention include those listed in the following table, which shows the CTFA designation (Polysorbate number), identity of the fatty acid used to produce the material and the number of moles of ethylene oxide reacted with each mole of ester. Compositions identified with an asterisk are predominately triesters.

| Polysorbate | Acid | Moles EtO |
|---|---|---|
| 20 | Lauric | 20 |
| 21 | Lauric | 4 |
| 40 | Palmitic | 20 |
| 60 | Stearic | 20 |
| 61 | Stearic | 4 |
| 65* | Stearic | 20 |
| 80 | Oleic | 20 |
| 81 | Oleic | 5 |
| 85* | Oleic | 20 |

In general, Polysorbate surfactants will be present in a formulation at about 50 to 500 µg /mL. When the surfactant concentration is below about 20 µg/mL, the particles tend to form cakes which are not easily redispersed.

Suitable surfactants include polyether glycols such as Pluronic^{®} F-68 (Poloxamer 188 a block copolymer of ethylene glycol and propylene glycols), Pluronic^{®} F87 (Poloxamer 237), Pluronic^{®} F108 (Poloxamer 338), Pluronic^{®} F127 (Poloxamer 407) and the like. Preferably, Pluronic^{®} F-68 is used. According to BASF Corporation's Technical Bulletin (1995), Pluronic^{®} is a registered tradename for BASF Corporation's block copolymers of ethylene oxide and propylene oxide represented by the chemical structure HO(C₂H₄O)ₐ(C₃H₆O)_{b}(C₂H₄O)ₐH wherein for: (a) Pluronic^{®} F-68, a is 80 and b is 27; (b) Pluronic^{®} F87, a is 64 and b is 37; (c) Pluronic^{®} F108, a is 141 and b is 44; and Pluronic^{®} F127, a is 101 and b is 56. The average molecular weights for these block copolymers are: (a) Pluronic^{®} F-68, 8400; (b) Pluronic^{®} F87, 7700; (c) Pluronic^{®} F108, 14600; and Pluronic^{®} F127, 12600.

Poloxamer surfactants are used at concentrations similar to those for the Polysorbates, although certain members are useful at concentrations up to about 1 mg/mL.

In general, the chosen surfactant should not materially increase the viscosity of the suspension formulation, since the efficiency of the nebulization process is particularly sensitive to viscosity. Many nonionic surfactants are useful for preparing inhalation and/or injectable drug formulations, and any of these should be suitable for use in the present invention.

The formulations further include a soluble salt. This salt performs at least two functions: it minimizes the effects of the inhaled formulation on the normal cell fluid balance of airway cells and also stabilizes the suspension of medicament. For the first function, it is preferred to use sufficient salt concentrations to render the formulation isotonic; sodium chloride and potassium chloride are preferred for this purpose. It has been found that adequate suspension stability is produced by isotonic concentrations (i.e., about 0.9 weight percent) of sodium chloride, although concentrations about 0.2 to about 2 weight percent are useful. Any physiologically compatible alkali metal or alkaline earth metal soluble salt can be used in the present invention.

Optionally, the formulations will contain a pH buffer, to maintain the formulation pH between about 3 and about 7. It has been found that stability of the drug (as measured by the absence of degradation reaction products) in suspension is improved by maintaining pH conditions below about 6. For reasons of tissue compatibility, excessively acidic products are not desired, so the pH should not be made to be below about 3. Some experimentation may be needed to qualify specific buffers for use in the invention: phosphate buffers in concentrations of 1 to 50 millimolar do not appear to adequately prevent caking of the particulates in the suspension when there is no added soluble salt. A citrate-citric acid buffer, maintaining pH between about 4 and about 5, has been used with particularly good effect for both maintaining pH during storage and preventing any particulate caking in the absence of soluble salts.

The citrate-citric acid buffer may be present in suspension formulations at concentrations at least about 2 and up to about 50 millimolar. While the literature has some reports of cough being induced by such buffer systems, this seems to occur primarily at the 150-200 millimolar level, although one report attributed cough to only a 35 millimolar concentration.

Sterility or adequate antimicrobial preservation of the final packaged formulation is needed for patient protection. The use of antimicrobial preservatives is less desirable, since certain of these have been associated with adverse clinical effects, such as bronchospasm. Alternative processes which may be considered for achieving sterility usually will not include sterilization steps for the micronized drug substance or formulation, since it has been found that the drug undergoes degradation under the influence of gamma-ray irradiation and sterilizing heat conditions. Sterilization by filtration ordinarily will not be feasible, due to the suspension nature of the formulation. Thus, it is preferred to produce the pharmacologically active agent, such asmometasone furoate monohydrate under sterile conditions, conduct the drug micronization in a sterile environment, and perform a sterile packaging operation.

Methods are known for reducing particle sizes into the micrometer range, including mechanical milling, application of ultrasonic energy and other techniques. Mechanical milling frequently generates high surface temperatures on the particles, and this is undesirable for Mometasone Furoate Monohydrate, a long acting beta agonist and/or Glycopyrrolate which tends to lose some part of its hydration under the influence of high temperatures. Ultrasonic techniques are quite slow in their action, generally requiring very long processing times, but are capable of producing acceptable suspensions.

Suspensions of drug particles can rapidly undergo particulate size reduction when subjected to "jet milling" (high pressure particle in liquid milling) techniques. A presently preferred jet milling procedure for producing the formulations of the invention involves the use of the "Microfluidizer" system sold by Microfluidics International Corporation of Newton, Mass., U.S.A. This device divides a fluid stream, flowing under high pressures (up to about 40,000 pounds per square inch, or 2.76.times.10⁸ newton/meter²), between two separate microchannel paths and then recombines them from generally perpendicular directions to create very high shear, impact and cavitation forces. By continuously recirculating suspensions through the system for a predetermined time period, it is possible to reproducibly create desired distributions of micron- and submicron-sized particles. Since the particles are always completely surrounded by liquid, their surfaces will not develop high temperatures under the influence of the size reduction forces, and the hydration water in the drug crystals will remain intact. Other useful equipment which utilizes related technology is available from Avestin Inc., Ottawa, Ontario, Canada.

In another embodiment, the pharmaceutically active agents may be solubilized utilizing cyclodextrins. One such cyclodextrin, for instance, is sold under the name Captisol. More specifically, Captisol® is a sulfobutyl ether derivative of beta-cyclodextrin with an average of seven sulfobutyl ether groups per cyclodextrin molecule. Because of the very low pKa of the sulfonic acid groups, Captisol® carries multiple negative charges at physiologically compatible pH values. The four-carbon butyl chain coupled with repulsion of the end group negative charges allows for an extension" of the cyclodextrin cavity. This often results in an increased possibility of inclusion complexation of the active agents with a relatively large molecular volume than has been demonstrated with other modified cyclodextrins. In addition, these derivatives impart exceptional solubility to the molecule.. The product is available Cydex, Inc. of Overland Park, Kansas. It may be prepared in accordance with the procedures set forth in International Patent Application WO 91/11172, incorporated by reference in it's entirety.

The foregoing descriptions of various embodiments of the invention are representative of various aspects of the invention, and are not intended to be exhaustive or limiting to the precise forms disclosed. Many modifications and variations undoubtedly will occur to those having skill in the art. It is intended that the scope of the invention shall be fully defined solely by the appended claims.

Preferred embodiments of the present invention are described below and are referred to as embodiments E1 to E37.
E1. A medicament comprising, separately or together, (A) an anticholinergic, (B) a corticosteroid, and (C) a long acting beta agonist, for simultaneous or sequential administration in the prevention or treatment of a respiratory, inflammatory or obstructive airway disease, each of (A), (B), and (C) being in an inhalable form.
E 2. The medicament of E 1 which is a pharmaceutical composition comprising an inhalable mixture of effective amounts of (A) and (B) and (C), optionally together with a pharmaceutically acceptable carrier.
E 3. The medicament of E2, which is an aerosol comprising a mixture of (A) and (B) and (C) in solution or dispersion in a propellant, or a combination of an aerosol containing (A) in solution or dispersion in a propellant with an aerosol containing (B) in solution or dispersion in a propellant with an aerosol containing (C) in solution or dispersion in a propellant.
E4. The medicament of E 3, in which (A) or (B) or (C), or (A) and (B) and (C), are in dispersion in the propellant, which is a halogen-substituted hydrocarbon.
E5. The medicament of E 4, in which (A) or (B) or (C), or each of (A) and (B) and (C), has an average particle diameter of up to 10 microns.
E 6. The medicament of E 3, which is a nebulizable composition comprising a dispersion of (A) and (B) and (C) in an aqueous, organic or aqueous/organic medium or a combination of a dispersion of (A) in said medium with a dispersion of (B) in said medium with a dispersion of (C) in said medium.
E7. The medicament of E2, which is a dry powder comprising finely divided (A) or (B) or (C), or finely divided (A) and (B) and (C), optionally together with a pharmaceutically acceptable carrier in finely divided form.
E8. The medicament of E 7, in which the carrier is present and is a saccharide.
E 9. The medicament of E 8, in which the carrier is lactose.
E 10. The medicament of E9, in which (A) or (B) or (C), or each of (A) and (B) and (C), has an average particle diameter up to 10 microns.
E 11. A medicament comprising, separately or together, (A) an anticholinergic, (B) a corticosteroid, and (C) a long acting beta agonist, for simultaneous or sequential administration in the prevention ortreatment of a respiratory, inflammatory or obstructive airway disease, each of (A), (B), and (C) being in inhalable form, wherein the anticholinergic is (R)-3-[2-hydroxy-2,2-(dithien-2-yl)acetoxy]-1-1[2-(phenyl)ethyl]-1-azoniabicyclo[2.2.2] octane or a pharmaceutically acceptable salt or hydrate thereof, wherein the corticosteroid is Mometasone Furoate or a pharmaceutically acceptable salt or hydrate thereof, and wherein the long acting beta agonist is Carmoterol, Indacaterol or a pharmaceutically acceptable salt or hydrate of any of the above, or a combination of two or more of the above.
E 12. A medicament comprising, separately or together, (A) an anticholinergic, (B) a corticosteroid, and (C) a long acting beta agonist, for simultaneous or sequential administration in the prevention or treatment of a respiratory, inflammatory or obstructive airway disease, each of (A), (B), and (C) being in inhalable form, wherein the anticholinergic is Glycopyrrolate or a pharmaceutically acceptable salt or hydrate thereof, wherein the corticosteroid is Mometasone Furoate or a pharmaceutically acceptable salt or hydrate thereof, and wherein the long acting beta agonist is Carmoterol, Indacaterol or a pharmaceutically acceptable salt or hydrate of any of the above, or a combination of two or more of the above.
E 13. The medicament of E 1, wherein the anticholinergic is (R)-3-[2-hydroxy-2,2-(dithien-2-yl)acetoxy]-1-1 [2-(phenyl)ethyl]-1-azoniabicyclo[2.2.2] octane, Glycopyrrolate, Ipratropium Bromide, Oxitropium Bromide, Atropine Methyl Nitrate, Atropine Sulfate, Ipratropium, Belladonna Extract, Scopolamine, Scopolamine Methobromide, Methscopolamine, Homatropine Methobromide, Hyoscyamine, Isopriopramide, Orphenadrine, Benzalkonium Chloride, Tiotropium Bromide, GSK202405, or a pharmaceutically acceptable salt or hydrate of any of the above, or a combination of two or more of the above.
E 14. The medicament of E 1, wherein the anticholinergic is (R)-3-[2-hydroxy-2,2-(dithien-2-yl)acetoxy]-1-1 [2-(phenyl)ethyl]-1-azoniabicyclo[2.2.2] octane or a pharmaceutically acceptable salt or hydrate thereof.
E 15. The medicament of E1, wherein the anticholinergic is Glycopyrrolate or a pharmaceutically acceptable salt or hydrate thereof.
E 16. The medicament of E 1, wherein the corticosteroid is Mometasone Furoate; Beclomethasone Dipropionate; Budesonide; Fluticasone; Dexamethasone; Flunisolide; Triamcinolone; (22R)-6.alpha.,9.alpha.-difluoro-11.beta.,21-dihydroxy-16.alpha.,17.alpha. -propylmethylenedioxy-4-pregnen-3,20-dione, Tipredane, GSK685698, GSK799943 or a pharmaceutically acceptable salt or hydrate of any of the above, or a combination of two or more of the above.
E 17. The medicament of E 14, wherein the corticosteroid is Mometasone Furoate or a pharmaceutically acceptable salt or hydrate thereof.
E 18. The medicament of E15, wherein the corticosteroid is Mometasone Furoate or a pharmaceutically acceptable salt or hydrate thereof.
E 19. The medicament of E 1, wherein the long acting beta agonist is Carmoterol, Indacaterol, TA-2005, Albuterol, Terbutaline, Salmeterol, Bitolterol, Formoterol, Fenoterol, Metaprotenerol, GSK159802, GSK642444, GSK159797, GSK597901, GSK678077, or a pharmaceutically acceptable salt or hydrate of any of the above, or a combination of two or more of the above.
E 20. The medicament of E 1, wherein the long acting beta agonist is Carmoterol or a pharmaceutically acceptable salt or hydrate thereof.
E 21. The medicament of E 1, wherein the long acting beta agonist is Indacaterol or a pharmaceutically acceptable salt or hydrate thereof.
E 22. The medicament of E 20, wherein the corticosteroid is Mometasone Furoate or a pharmaceutically acceptable salt or hydrate thereof.
E23. The medicament of E 21, wherein the corticosteroid is Mometasone Furoate or a pharmaceutically acceptable salt or hydrate thereof.
E 24. The medicament of E1, in admixture with a polyanionic beta-cyclodextrin derivative with about one to about seven sodium sulfonate groups separated from the lipophilic cavity of the polyanionic beta-cyclodextrin derivative by at least one butyl ether spacer group.
E25. The medicament of E 1, further comprising, (D) a phosphodiesterase IV inhibitor, wherein (D) is in an inhalable form.
E26. The medicament of E25, wherein the phosphodiesterase IV inhibitor is Cilomilast, Roflumilast, Tetomilast, 1-[[5-(1(S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester or a pharmaceutically acceptable salt or hydrate of any of the above, or a combination of two or more of the above.
E27. The medicament of E 25, wherein the phosphodiesterase IV inhibitor is 1-[[5-(1 (S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester or a pharmaceutically acceptable salt or hydrate thereof.
E 28. The medicament of E 25, wherein the phosphodiesterase IV inhibitor is a xinafoate salt of 1-[[5-(1 (S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester.
E 29. The medicament of E 25, wherein the anticholinergic is Glycopyrrolate or a pharmaceutically acceptable salt or hydrate thereof, wherein the corticosteroid is Mometasone Furoate or a pharmaceutically acceptable salt or hydrate thereof, wherein the long acting beta agonist is Carmoterol, Indacaterol or a pharmaceutically acceptable salt or hydrate of any of the above, or a combination of two or more of the above, and wherein the phosphodiesterase IV inhibitor is a xinafoate salt of 1-[[5-(1 (S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester.
E 30. The medicament of E25, wherein the anticholinergic is (R)-3-[2-hydroxy-2,2-(dithien-2-yl)acetoxy]-1-1 [2-(phenyl)ethyl]-1-azoniabicyclo[2.2.2] octane or a pharmaceutically acceptable salt or hydrate thereof, wherein the corticosteroid is Mometasone Furoate or a pharmaceutically acceptable salt or hydrate thereof, wherein the long acting beta agonist is Carmoterol, Indacaterol or a pharmaceutically acceptable salt or hydrate of any of the above, or a combination of two or more of the above, and wherein the phosphodiesterase IV inhibitor is a xinafoate salt of 1-[[5-(1(S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester.
E31. The medicament of E25, in admixture with a polyanionic beta-cyclodextrin derivative with about one to about seven sodium sulfonate groups separated from the lipophilic cavity of the polyanionic beta-cyclodextrin derivative by at least one butyl ether spacer group.
E 32. A medicament comprising phosphodiesterase IV inhibitor in an inhalable form.
E 33. The medicament of E 32, wherein the phosphodiesterase IV inhibitor is Cilomilast, Roflumilast, Tetomilast, 1-[[5-(1(S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester or a pharmaceutically acceptable salt or hydrate of any of the above, or a combination of two or more of the above.
E 34. The medicament of E 32, wherein the phosphodiesterase IV inhibitor is 1-[[5-(1 (S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester or a pharmaceutically acceptable salt or hydrate thereof.
E 35. The medicament of E32, wherein the phosphodiesterase IV inhibitor is a xinafoate salt of 1-[[5-(1(S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester.
E 36. A pharmaceutical kit comprising (A) as defined in E1, and (B) as defined in E1, and (C) as defined in E 1, in separate unit dosage forms, said forms being suitable for administration of (A) and (B) and (C) in effective amounts, together with one or more inhalation devices for administration of (A) and (B) and (C), and instructions for administering (A) and (B) and (C).
E37. A pharmaceutical kit comprising (A) as defined in E 25, and (B) as defined in E25, and (C) as defined in E 25, and (D) as defined in E 25, in separate unit dosage forms, said forms being suitable for administration of (A) and (B) and (C) and (D) in effective amounts, together with one or more inhalation devices for administration of (A) and (B) and (C) and (D), and instructions for administering (A) and (B) and (C) and (D).

## Claims

1. A medicament comprising a phosphodiesterase IV inhibitor in an inhalable form.

2. The medicament of claim 1, wherein the phosphodiesterase IV inhibitor is 1-[[5-(1 (S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester, Cilomilast, Roflumilast, Tetomilast, or a pharmaceutically acceptable salt or hydrate of any of the above, or a combination of two or more of the above.

3. The medicament of claim 2, wherein the phosphodiesterase IV inhibitor is 1-[[5-(1 (S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester or a pharmaceutically acceptable salt or hydrate thereof.

4. The medicament of claim 2, wherein the phosphodiesterase IV inhibitor is a xinafoate salt of 1-[[5-(1(S)-aminoethyl)-2-[8-methoxy-2-(trifluoromethyl)-5-quinolinyl]-4 oxazolyl]carbonyl]-4(R)-[(cyclopropylcarbonyl)amino]-L-proline, ethyl ester.

5. The medicament of any one of the preceding claims further comprising, separately or together, (A) an anticholinergic, (B) a corticosteroid, and (C) a long acting beta agonist, each of (A), (B), and (C) being in an inhalable form.

6. The medicament of any one of the preceding claims, for use in the treatment or prevention of a respiratory, inflammatory or obstructive airway disease.

7. A pharmaceutical kit comprising (A) as defined in claim 5, (B) as defined in claim 5, (C) as defined in claim 5, and (D) a phosphodiesterase IV inhibitor in an inhalable form, in separate unit dosage forms, said forms being suitable for administration of (A) and (B) and (C) and (D) in effective amounts, together with one or more inhalation devices for administration of (A) and (B) and (C) and (D), and instructions for administering (A) and (B) and (C) and (D).

8. The kit of claim 7, wherein the phosphodiesterase IV inhibitor is as defined in any one of claims 2 to 4.

9. The kit of claim 7, wherein the phosphodiesterase IV inhibitor is a xinafoate salt of 1-[[5-(1 (S)-aminoethyl)-2-[8-methoxy-2-(trifluoromethyl)-5-quinolinyl]-4 oxazolyl]carbonyl]-4(R)-[(cyclopropylcarbonyl)amino]-L-proline, ethyl ester.

10. The kit of any one of claims 7 to 9, for use in the treatment or prevention of a respiratory, inflammatory or obstructive airway disease.

11. A compound which is a xinafoate salt of 1-[[5-(1(S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester.

12. The compound of claim 11 for use in the treatment or prevention of a respiratory, inflammatory or obstructive airway disease.
